# EUROPEAN PATENT SPECIFICATION

(11) **EP 3 888 557 B1**
(45) Date of publication and mention of the grant of the patent: **01.05.2024**
(21) Application number: 19888703.6
(22) Date of filing: 17.10.2019
(51) Int. Cl.: A61B 8/06, A61B 8/14

(54) **ULTRASONIC DIAGNOSTIC DEVICE AND METHOD FOR CONTROLLING ULTRASONIC DIAGNOSTIC DEVICE**
ULTRASCHALLDIAGNOSEVORRICHTUNG UND VERFAHREN ZUR STEUERUNG DER ULTRASCHALLDIAGNOSEVORRICHTUNG
DISPOSITIF DE DIAGNOSTIC ULTRASONORE ET PROCÉDÉ DE COMMANDE DE DISPOSITIF DE DIAGNOSTIC ULTRASONORE

(30) Priority: 30.11.2018 JP 2018225216
(43) Date of publication of application: 06.10.2021
(73) Proprietor: FUJIFILM Corporation, Tokyo 106-8620 (JP)
(72) Inventor: YAMAMOTO Katsuya, kami-gun, Kanagawa 258-8538 (JP)
(74) Representative: Hughes, Andrea Michelle
(86) International application number: PCT/JP2019/040920
(87) International publication number: WO 2020/110500

(56) References cited:
- WO-A1-2014/045572
- JP-A- 2002 052 026
- JP-A- 2004 344 564
- JP-A- 2004 344 564
- JP-A- 2006 087 696

## Description

### BACKGROUND OF THE INVENTION

### 1. Field of the Invention

The invention relates to an ultrasound diagnostic apparatus and a control method of the ultrasound diagnostic apparatus, and particularly to an ultrasound diagnostic apparatus and a control method of the ultrasound diagnostic apparatus which measure a blood flow rate in a subject.

### 2. Description of the Related Art

In the related art, an ultrasound diagnostic apparatus has been known as an apparatus for obtaining an image of the inside of a subject. The ultrasound diagnostic apparatus generally comprises an ultrasound probe comprising a transducer array in which a plurality of elements are arranged. In a state where the ultrasound probe is in contact with a body surface of the subject, an ultrasound beam is transmitted toward the inside of the subject from the transducer array and an ultrasound echo from the subject is received by the transducer array so that element data is acquired. Further, the ultrasound diagnostic apparatus electrically processes the obtained element data to generate an ultrasound image of the corresponding site of the subj ect.

For example, JP2002-052026A discloses an ultrasound diagnostic apparatus which installs a Doppler gate on a B-mode image, and searches a search region including a center point of the Doppler gate to detect a vascular wall and to calculate a blood vessel diameter. The ultrasound diagnostic apparatus calculates a blood flow velocity in a blood vessel on the basis of Doppler data in the Doppler gate, and measures the blood flow rate in the blood vessel by using the calculated blood flow velocity and blood vessel diameter.

JP 2004344564 A discloses a cardiac output measurement device and method wherein an enlarged image is used to set a sample gate.

### SUMMARY OF THE INVENTION

In a case where the blood flow rate in the subject is measured, it is preferable that the user checks both the B-mode image and Doppler data which are used for the measurement, such that appropriate measurement is performed, for example, whether the Doppler gate is placed at an appropriate position.

Normally, in a case where the measurement of the blood flow rate is performed using the ultrasound diagnostic apparatus in the related art as disclosed in JP2002-052026A, although an ultrasound image in which a wide region including not only the blood vessel region but also the peripheral tissues is shown is used, since the blood vessel region as the measurement target is shown relatively small compared to the display region of the B-mode image, there is a problem in that it is difficult for the user to clearly check the blood vessel region on the B-mode image and an error occurs in the measurement position or the like so that the measurement accuracy is decreased.

The present invention has been made in order to solve such a problem in the related art, and an object of the present invention is to provide an ultrasound diagnostic apparatus and a control method of the ultrasound diagnostic apparatus in which the user can clearly check the blood vessel region on the B-mode image and which can improve the measurement accuracy of the blood flow rate.

In order to achieve the object, an ultrasound diagnostic apparatus according to an aspect of the invention comprises a gate setting unit that sets a Doppler gate in a blood vessel region by performing an image analysis on a B-mode image in which at least the blood vessel region is imaged; a Doppler processing unit that generates a Doppler waveform image on the basis of Doppler data in the Doppler gate; a display unit that displays the B-mode image and the Doppler waveform image; and an image enlargement unit that, in a case where both the B-mode image and the Doppler waveform image are frozen by a user, displays an enlarged B-mode image in which the blood vessel region including the Doppler gate is enlarged, on the display unit.

The Doppler processing unit calculates a blood flow velocity on the basis of the Doppler data in the Doppler gate, and the ultrasound diagnostic apparatus further comprises a vascular wall detection unit that detects an anterior vascular wall and a posterior vascular wall by performing the image analysis on the enlarged B-mode image; a cross-sectional area calculation unit that calculates a cross-sectional area of a blood vessel on the basis of the anterior vascular wall and the posterior vascular wall detected by the vascular wall detection unit; and a blood flow rate measurement unit that measures a blood flow rate on the basis of the cross-sectional area of the blood vessel calculated by the cross-sectional area calculation unit and the blood flow velocity calculated by the Doppler processing unit.

In this case, it is preferable that the image enlargement unit displays the blood flow rate measured by the blood flow rate measurement unit together with the enlarged B-mode image on the display unit.

The image enlargement unit may enlarge the blood vessel region such that a center position of the Doppler gate is a center position of the enlarged B-mode image.

Alternatively, the image enlargement unit may enlarge the blood vessel region such that a gate width of the Doppler gate in the enlarged B-mode image has a predetermined value.

Alternatively, the image enlargement unit may enlarge the blood vessel region such that a distance between the anterior vascular wall and the posterior vascular wall in the enlarged B-mode image has a predetermined value.

Further, the display unit may have a B-mode image display region for displaying the B-mode image, and the image enlargement unit may hold a plurality of predetermined magnifications which are different from each other, and enlarge the blood vessel region by the maximum magnification where the anterior vascular wall and the posterior vascular wall are included in the B-mode image display region, among the plurality of predetermined magnifications.

Alternatively, the Doppler processing unit may generate the Doppler waveform image on the basis of the Doppler data in the Doppler gate, the display unit may have a B-mode image display region for displaying the B-mode image and a Doppler waveform image display region for displaying the Doppler waveform image, and the ultrasound diagnostic apparatus may further comprise a size ratio change unit that changes a size ratio of the B-mode image display region and the Doppler waveform image display region on the basis of a size of the enlarged B-mode image.

Further, in a case where both the B-mode image and the Doppler waveform image are frozen by the user, the blood flow rate may be automatically measured by the blood flow rate measurement unit.

A control method of an ultrasound diagnostic apparatus according to another aspect of the invention is defined by the features of claim 10.

According to the invention, since there are provided the gate setting unit that sets the Doppler gate in the blood vessel region by performing the image analysis on the B-mode image in which at least the blood vessel region is imaged; the Doppler processing unit that generates the Doppler waveform image on the basis of the Doppler data in the Doppler gate; the display unit that displays the B-mode image and the Doppler waveform image; and the image enlargement unit that, in a case where both the B-mode image and the Doppler waveform image are frozen by the user, displays the enlarged B-mode image in which the blood vessel region including the Doppler gate is enlarged, on the display unit, it is possible for the user to clearly check the blood vessel region on the B-mode image and it is possible to improve the measurement accuracy of the blood flow rate.

### BRIEF DESCRIPTION OF THE DRAWINGS

Fig. 1 is a block diagram illustrating a configuration of an ultrasound diagnostic apparatus according to a first embodiment of the invention.
Fig. 2 is a block diagram illustrating an internal configuration of a reception unit in the first embodiment of the invention.
Fig. 3 is a block diagram illustrating an internal configuration of a B-mode processing unit in the first embodiment of the invention.
Fig. 4 is a block diagram illustrating an internal configuration of a Doppler processing unit in the first embodiment of the invention.
Fig. 5 is a block diagram illustrating an internal configuration of a vascular wall detection unit in the first embodiment of the invention.
Fig. 6 is a diagram schematically illustrating a designation point designated by a user in the first embodiment of the invention.
Fig. 7 is a diagram schematically illustrating a method of detecting a vascular wall by the vascular wall detection unit in the first embodiment of the invention.
Fig. 8 is a diagram illustrating a B-mode image in which a Doppler gate is set in the first embodiment of the invention.
Fig. 9 is a diagram illustrating a B-mode image and a Doppler waveform image in the first embodiment of the invention.
Fig. 10 is a diagram illustrating an enlarged B-mode image and a Doppler waveform image in the first embodiment of the invention.
Fig. 11 is a flowchart illustrating an operation of the ultrasound diagnostic apparatus according to the first embodiment of the invention.
Fig. 12 is a flowchart illustrating an operation of automatically measuring a blood flow rate in the first embodiment of the invention.
Fig. 13 is a diagram illustrating a display example of the blood flow rate measured in the first embodiment of the invention.
Fig. 14 is a diagram illustrating a B-mode image and a Doppler waveform image in a second embodiment of the invention.
Fig. 15 is a diagram illustrating an enlarged B-mode image and a Doppler waveform image in the second embodiment of the invention.

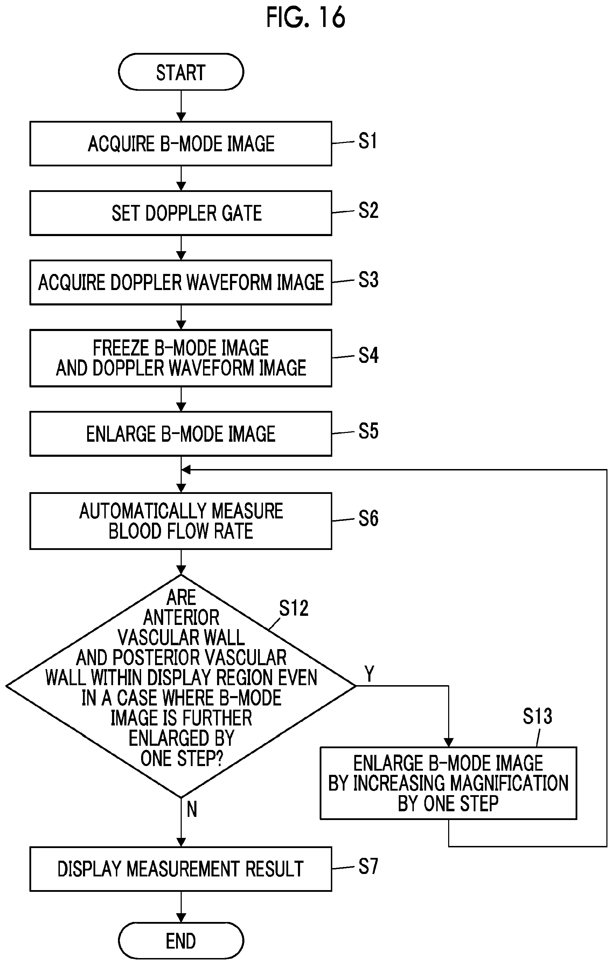

Fig. 16 is a flowchart illustrating an operation of an ultrasound diagnostic apparatus according to a third embodiment of the invention.
Fig. 17 is a diagram illustrating an enlarged B-mode image and a Doppler waveform image in the third embodiment of the invention.
Fig. 18 is a diagram illustrating the Doppler waveform image and the enlarged B-mode image in which the blood vessel region is enlarged at the maximum magnification where an anterior vascular wall and a posterior vascular wall are included, in the third embodiment of the invention.
Fig. 19 is a block diagram illustrating a configuration of an ultrasound diagnostic apparatus according to a fourth embodiment of the invention.
Fig. 20 is a diagram illustrating a B-mode image and a Doppler waveform image in the fourth embodiment of the invention.
Fig. 21 is a diagram illustrating a B-mode image display region and a Doppler waveform image display region of which a size ratio of a display region is changed, in the fourth embodiment of the invention.
Fig. 22 is a diagram illustrating an enlarged B-mode image and a Doppler waveform image in a modification example of the fourth embodiment of the invention.
Fig. 23 is a block diagram illustrating a configuration of an ultrasound diagnostic apparatus according to a fifth embodiment of the invention.

### DESCRIPTION OF THE PREFERRED EMBODIMENTS

Hereinafter, embodiments of the invention will be described with reference to the accompanying drawings.

The description of constituents described below is given on the basis of the representative embodiment of the invention, but the invention is not limited to such an embodiment.

In the present specification, a numerical range represented using "to" means a range including the numerical values before and after "to" as a lower limit value and an upper limit value.

In addition, in the present specification, the terms "perpendicular" and "parallel" include a range of error allowed in the technical field to which the invention belongs. For example, the terms "perpendicular" and "parallel" mean a range less than ±10° with respect to the strict perpendicular or parallel, and the error with respect to the strict perpendicular or parallel is preferably 5° or less, and more preferably 3° or less.

In the present specification, the terms "same" and "identical" include an error range generally allowed in the technical field. Further, in the present specification, in a case of referring to "all", "any", or "whole surface", the term includes an error range generally allowed in the technical field in addition to a case of 100%, and includes, for example, a case of 99% or more, a case of 95% or more, or a case of 90% or more.

### First Embodiment

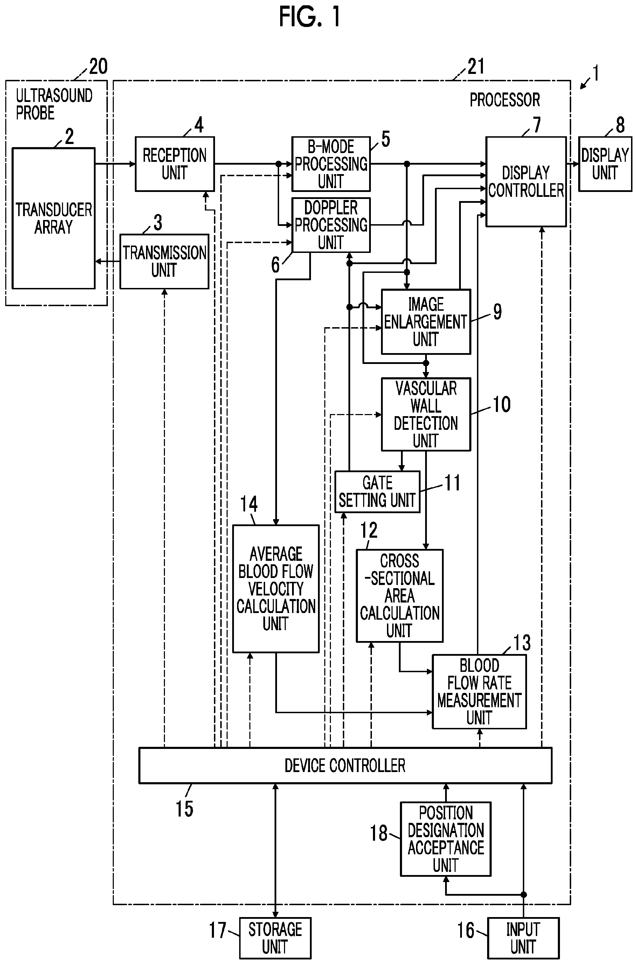

Fig. 1 illustrates a configuration of an ultrasound diagnostic apparatus 1 according to a first embodiment of the invention. As illustrated in Fig. 1, the ultrasound diagnostic apparatus 1 comprises a transducer array 2, and each of a transmission unit 3 and a reception unit 4 is connected to the transducer array 2. A B-mode processing unit 5 and a Doppler processing unit 6 are connected in parallel to the reception unit 4, and a display unit 8 is connected to the B-mode processing unit 5 and the Doppler processing unit 6 via a display controller 7.

An image enlargement unit 9 is connected to the B-mode processing unit 5, and a vascular wall detection unit 10 is connected to the image enlargement unit 9. The B-mode processing unit 5 is also connected to the vascular wall detection unit 10. A gate setting unit 11 and a cross-sectional area calculation unit 12 are connected to the vascular wall detection unit 10. The gate setting unit 11 is connected to the Doppler processing unit 6 and the image enlargement unit 9. A blood flow rate measurement unit 13 is connected to the cross-sectional area calculation unit 12. In addition, an average blood flow velocity calculation unit 14 is connected to the Doppler processing unit 6, and the blood flow rate measurement unit 13 is connected to the average blood flow velocity calculation unit 14. Further, each of the image enlargement unit 9, the gate setting unit 11, and the blood flow rate measurement unit 13 is connected to the display controller 7.

In addition, a device controller 15 is connected to the transmission unit 3, the reception unit 4, the B-mode processing unit 5, the Doppler processing unit 6, the display controller 7, the image enlargement unit 9, the vascular wall detection unit 10, the gate setting unit 11, the cross-sectional area calculation unit 12, the blood flow rate measurement unit 13, and the average blood flow velocity calculation unit 14, and an input unit 16 and a storage unit 17 are connected to the device controller 15. Further, a position designation acceptance unit 18 is connected to the input unit 16, and the position designation acceptance unit 18 is connected to the device controller 15. Here, the device controller 15 and the storage unit 17 are connected so as to exchange information bidirectionally.

Further, the transducer array 2 is included in an ultrasound probe 20, and the transmission unit 3, the reception unit 4, the B-mode processing unit 5, the Doppler processing unit 6, the display controller 7, the image enlargement unit 9, the vascular wall detection unit 10, the gate setting unit 11, the cross-sectional area calculation unit 12, the blood flow rate measurement unit 13, the average blood flow velocity calculation unit 14, the device controller 15, and the position designation acceptance unit 18 constitute a processor 21.

The transducer array 2 of the ultrasound probe 20 illustrated in Fig. 1 has a plurality of transducers arranged in a one-dimensional or two-dimensional manner. According to a drive signal supplied from the transmission unit 3, each of the transducers transmits an ultrasonic wave and receives an ultrasound echo from a subject to output a signal based on the ultrasound echo. For example, each transducer is configured by forming electrodes at both ends of a piezoelectric body consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

The transmission unit 3 of the processor 21 includes, for example, a plurality of pulse generators, and the transmission unit 3 adjusts the amount of delay of each drive signal so that ultrasonic waves transmitted from the plurality of transducers of the transducer array 2 form an ultrasound beam on the basis of a transmission delay pattern selected according to the control signal from the device controller 15, and supplies the obtained signals to the plurality of transducers. Thus, in a case where a pulsed or continuous-wave voltage is applied to the electrodes of the transducers of the transducer array 2, the piezoelectric body expands and contracts to generate pulsed or continuous-wave ultrasonic waves from each transducer. From the combined wave of these ultrasonic waves, an ultrasound beam is formed.

The transmitted ultrasound beam is reflected by a target, for example, a site of the subject, and propagates toward the transducer array 2 of the ultrasound probe 20. The ultrasonic waves propagating toward the transducer array 2 in this manner are received by each transducer constituting the transducer array 2. In this case, each transducer constituting the transducer array 2 expands and contracts by receiving the propagating ultrasound echo to generate electrical signals, and outputs the electrical signals to the reception unit 4.

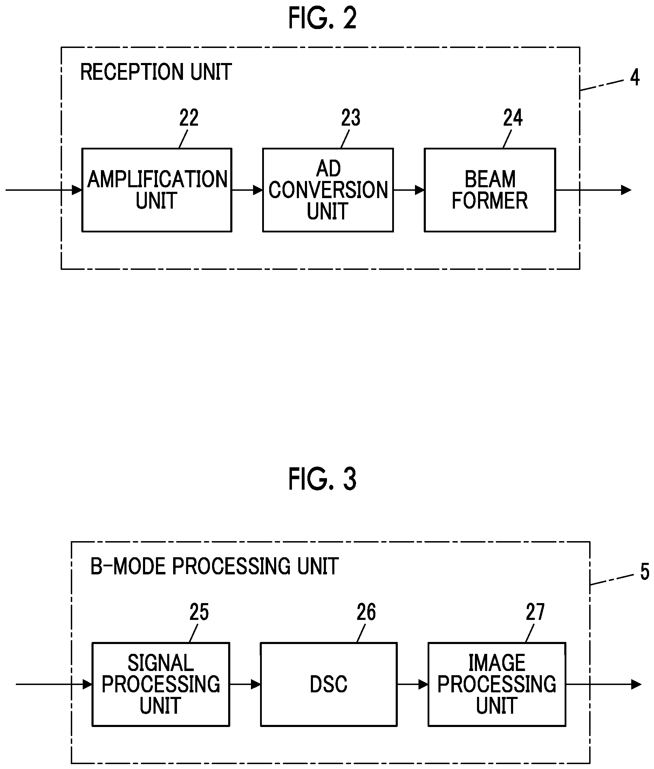

The reception unit 4 of the processor 21 processes the signals output from the transducer array 2 according to the control signals from the device controller 15. As illustrated in Fig. 2, the reception unit 4 has a configuration in which an amplification unit 22, an analog digital (AD) conversion unit 23, and a beam former 24 are connected in series.

The amplification unit 22 amplifies the signals input from each transducer constituting the transducer array 2, and transmits the amplified signals to the AD conversion unit 23. The AD conversion unit 23 converts the signals transmitted from the amplification unit 22 into digital data, and transmits the data to the beam former 24. The beam former 24 performs so-called reception focusing processing in which addition is performed by giving delays to respective pieces of data converted by the AD conversion unit 23 according to a sound speed distribution or a sound speed set on the basis of a reception delay pattern selected according to the control signals from the device controller 15. Through the reception focusing processing, a reception signal in which each piece of data converted by the AD conversion unit 23 is phased and added and the focus of the ultrasound echo is narrowed is acquired.

As illustrated in Fig. 3, the B-mode processing unit 5 of the processor 21 has a configuration in which a signal processing unit 25, a digital scan converter (DSC) 26, and an image processing unit 27 are sequentially connected in series.

The signal processing unit 25 generates a B-mode image signal, which is tomographic image information regarding tissues inside the subject, by performing, on reception data generated by the reception unit 4, correction of the attenuation due to the distance according to the depth of the reflection position of the ultrasonic wave and then performing envelope detection processing.

The DSC 26 converts (raster conversion) the B-mode image signal generated by the signal processing unit 25 into an image signal according to a normal television signal scanning method.

The image processing unit 27 performs various kinds of necessary image processing such as gradation processing on the B-mode image signal input from the DSC 26, and then outputs the B-mode image signal to the display controller 7.

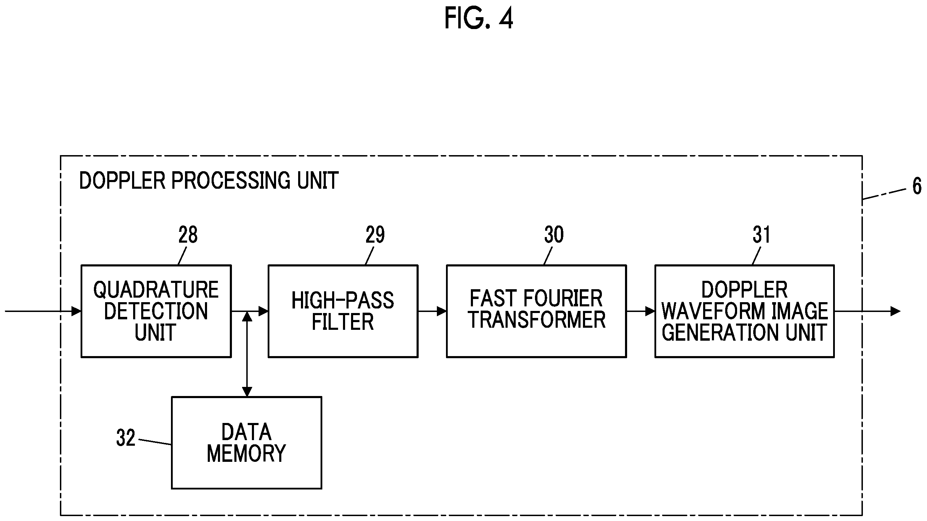

The Doppler processing unit 6 of the processor 21 calculates the blood flow velocity using a so-called pulse Doppler method and generates a Doppler waveform image. As illustrated in Fig. 4, the Doppler processing unit 6 has a configuration in which a quadrature detection unit 28, a high-pass filter 29, a fast Fourier transformer 30, and a Doppler waveform image generation unit 31 are sequentially connected in series and a data memory 32 is connected to an output terminal of the quadrature detection unit 28.

The quadrature detection unit 28 mixes the reception data generated by the reception unit 4 with a carrier signal having a reference frequency to perform quadrature detection on the reception data and converts the reception data into complex data.

The high-pass filter 29 functions as a so-called wall filter, and removes a frequency component derived from the motion of the body tissue inside the subject, from the complex data generated by the quadrature detection unit 28.

The fast Fourier transformer 30 performs a Fourier transform on the complex data of a plurality of sample points to perform frequency analysis, obtains the blood flow velocity, and generates a spectrum signal.

The Doppler waveform image generation unit 31 generates a Doppler waveform image by aligning the spectrum signals generated by the fast Fourier transformer 30 on a time axis and expressing the magnitude of each frequency component in brightness. In the Doppler waveform image, the lateral axis indicates a time axis, the vertical axis indicates a Doppler shift frequency, that is, a flow velocity, and the brightness of the waveform represents power in each frequency component.

Further, the data memory 32 saves the complex data converted from the reception data by the quadrature detection unit 28.

The device controller 15 of the processor 21 controls each unit of the ultrasound diagnostic apparatus 1 on the basis of a program stored in advance in the storage unit 17 or the like and the user's operation through the input unit 16.

The display controller 7 of the processor 21 performs predetermined processing on the B-mode image signal generated by the B-mode processing unit 5 and the Doppler waveform image signal generated by the Doppler processing unit 6, and displays the B-mode image and the Doppler waveform image on the display unit 8, under the control of the device controller 15.

The display unit 8 of the ultrasound diagnostic apparatus 1 displays the generated image under the control of the display controller 7, and includes, for example, a display device such as a liquid crystal display (LCD).

The input unit 16 of the ultrasound diagnostic apparatus 1 is for the user to perform an input operation, and can be configured to comprise a keyboard, a mouse, a trackball, a touchpad, a touch panel, and the like.

The position designation acceptance unit 18 of the processor 21 accepts a position designation of a blood vessel region by the user through the input unit 16, on the B-mode image displayed on the display unit 8. For example, in a case where the input unit 16 is configured by the touch panel, the position designation acceptance unit 18 can accept the position designation of the blood vessel region which is touched by the user's finger, a stylus pen, or the like.

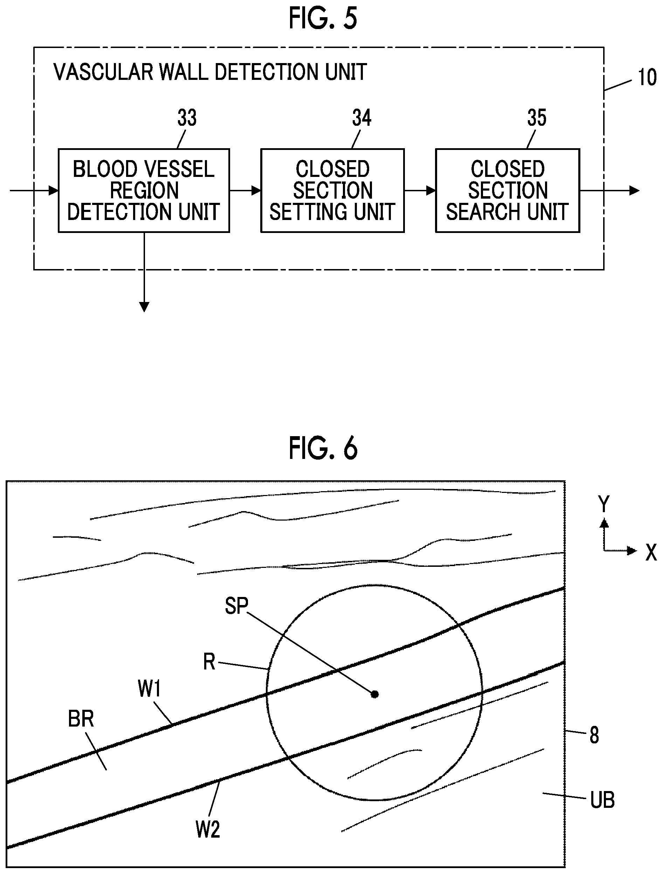

The vascular wall detection unit 10 of the processor 21 detects an anterior vascular wall and a posterior vascular wall by performing the image analysis on the B-mode image, on the basis of the position designation of the blood vessel region by the user which is accepted by the position designation acceptance unit 18. As illustrated in Fig. 5, the vascular wall detection unit 10 has a configuration in which a blood vessel region detection unit 33, a closed section setting unit 34, and a closed section search unit 35 are connected in series.

Here, an upper vascular wall of the vascular walls on the B-mode image, that is, a vascular wall on a shallow portion side which is close to the body surface of the subject with which the ultrasound probe 20 is in contact is called the anterior vascular wall, and a lower vascular wall of the vascular walls on the B-mode image, that is, a vascular wall on a deep portion side which is far from the body surface of the subject with which the ultrasound probe 20 is in contact is called the posterior vascular wall. For example, for convenience, as illustrated in Fig. 6, in the screen of the display unit 8, in a case where a direction extending horizontally is set as an X direction and a direction extending vertically is set as a Y direction, of the vascular walls as the boundary of a blood vessel region BR on a B-mode image UB, an anterior vascular wall W1 is positioned on the upper side, that is, +Y direction side, and a posterior vascular wall W2 is positioned on the lower side, that is, -Y direction side.

The blood vessel region detection unit 33 of the vascular wall detection unit 10 performs the image analysis on the B-mode image UB generated by the B-mode processing unit 5 to detect the blood vessel region on the B-mode image UB. In this case, the blood vessel region detection unit 33 can detect the blood vessel region on the B-mode image UB using a known algorithm. For example, the blood vessel region detection unit 33 can store typical pattern data of the blood vessel region in advance as a template, calculate a similarity degree for the pattern data while searching the image using the template, and consider that the blood vessel region is present in a place where the similarity degree is equal to or greater than a threshold value and is the maximum.

For the calculation of the similarity degree, in addition to simple template matching, for example, a machine learning method described in Csurka et al.: Visual Categorization with Bags of Keypoints, Proc. of ECCV Workshop on Statistical Learning in Computer Vision, pp. 59-74 (2004) or a general image recognition method using deep learning described in Krizhevsk et al.: ImageNet Classification with Deep Convolutional Neural Networks, Advances in Neural Information Processing Systems 25, pp.1106-1114 (2012) can be used.

The closed section setting unit 34 of the vascular wall detection unit 10 sets a closed section which includes the position designated by the user via the position designation acceptance unit 18 and of which the blood vessel region detected by the blood vessel region detection unit 33 passes through the inside. For example, as illustrated in Fig. 6, the closed section setting unit 34 can set a circular closed section R centered on a designation position SP designated by the user through the input unit 16, on the B-mode image UB. In the example illustrated in Fig. 6, the blood vessel region BR passes through the inside of the closed section R. The closed section set by the closed section setting unit 34 is not limited to a circular shape illustrated in Fig. 6 and can have any shape as long as the closed section has a closed shape.

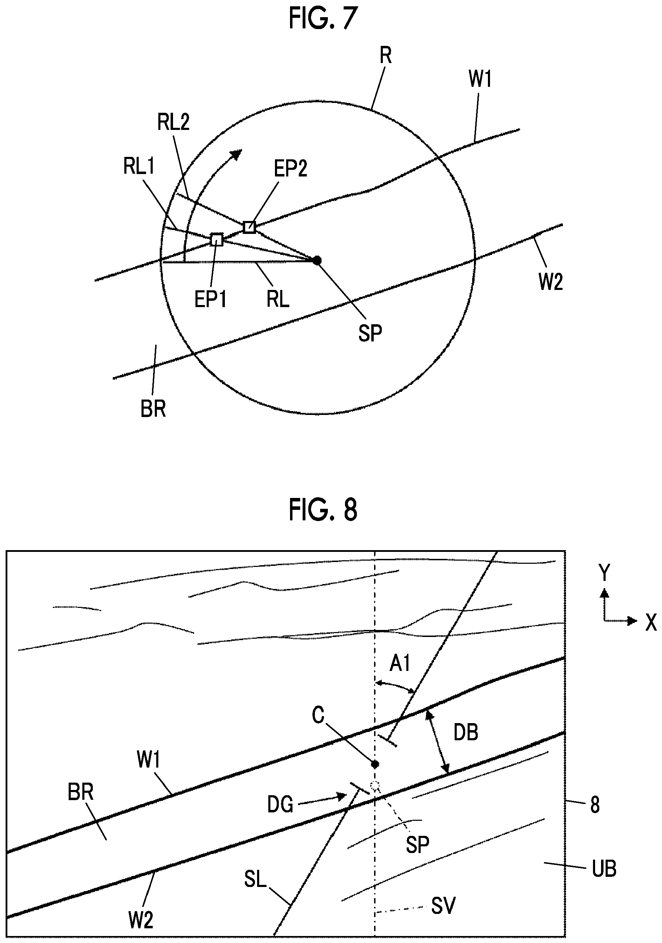

The closed section search unit 35 of the vascular wall detection unit 10 searches the inside of the closed section set by the closed section setting unit 34 to detect the anterior vascular wall W1 and the posterior vascular wall W2. In this case, for example, the closed section search unit 35 can search the inside of the closed section R using a method as disclosed in JP4749592B, and detect the anterior vascular wall W1 and the posterior vascular wall W2. Specifically, as illustrated in Fig. 7, the closed section search unit 35 searches for the B-mode intensity data outwards from the designation position SP along a search line RL connecting the designation position SP and the boundary of the closed section R, over an entire 360° range of the designation position SP designated by the user through the input unit 16, to detect a position where the amount of change in B-mode intensity is maximum, as the anterior vascular wall W1 or the posterior vascular wall W2. Here, as the B-mode intensity data, for example, a brightness value of the B-mode image signal can be used.

The example illustrated in Fig. 7 indicates a form in which the anterior vascular wall W1 and the posterior vascular wall W2 are searched for while the search line RL connecting the designation position SP and the boundary of the closed section R is scanned by a predetermined angle clockwise over 360° around the designation position SP, an edge point EP1 corresponding to the anterior vascular wall W1 on a search line RL1 is detected, and an edge point EP2 corresponding to the anterior vascular wall W1 on a search line RL2 is detected.

The gate setting unit 11 of the processor 21 sets a Doppler gate in the blood vessel region BR on the B-mode image UB on the basis of the anterior vascular wall W1 and the posterior vascular wall W2 detected by the vascular wall detection unit 10. In this case, the gate setting unit 11 can set the Doppler gate using a method disclosed in JP4749592B, for example. More specifically, as illustrated in Fig. 8, the gate setting unit 11 can detect a center position C of the blood vessel region BR on a vertical line SV passing through the designation position SP on the basis of the positions of the detected anterior vascular wall W1 and posterior vascular wall W2, and set a Doppler gate DG such that the center position C and the center of the Doppler gate DG overlap each other. In this case, for example, although not illustrated, the gate setting unit 11 can detect each of an intersection between the anterior vascular wall W1 and the vertical line SV and an intersection between the posterior vascular wall W2 and the vertical line SV, and detect the midpoint of the two detected intersections as the center position C.

Here, the vertical line SV is a virtual line extending along a vertical direction with respect to the display unit 8, that is, the Y direction. Further, the Doppler gate DG set by the gate setting unit 11 is inclined from the vertical line SV on the screen of the display unit 8 by a cursor steering angle A1, and the cursor steering angle A1 is equal to an inclination angle of a scan line SL passing through the center position C of the Doppler gate DG.

In a case where both the B-mode image UB and the Doppler waveform image are frozen by the user via the input unit 16 or the like, the image enlargement unit 9 of the processor 21 displays an enlarged B-mode image in which the blood vessel region BR including the Doppler gate DG is enlarged, on the display unit 8. Here, freezing the B-mode image UB means that, in a state where the B-mode images UB consecutively generated by the B-mode processing unit 5 are sequentially displayed on the display unit 8, the display of the B-mode image UB is paused and the one paused B-mode image UB is displayed on the display unit 8. Further, similar to the freezing of the B-mode image UB, freezing the Doppler waveform image means that, in a state where the Doppler waveform images consecutively generated by the Doppler processing unit 6 are sequentially displayed on the display unit 8, the display of the Doppler waveform image is paused and the one paused Doppler waveform image is displayed on the display unit 8. In a case of enlarging the blood vessel region BR, the image enlargement unit 9 enlarges the blood vessel region BR using the same magnification in the vertical direction and the lateral direction orthogonal to the vertical direction of the B-mode image UB, for example.

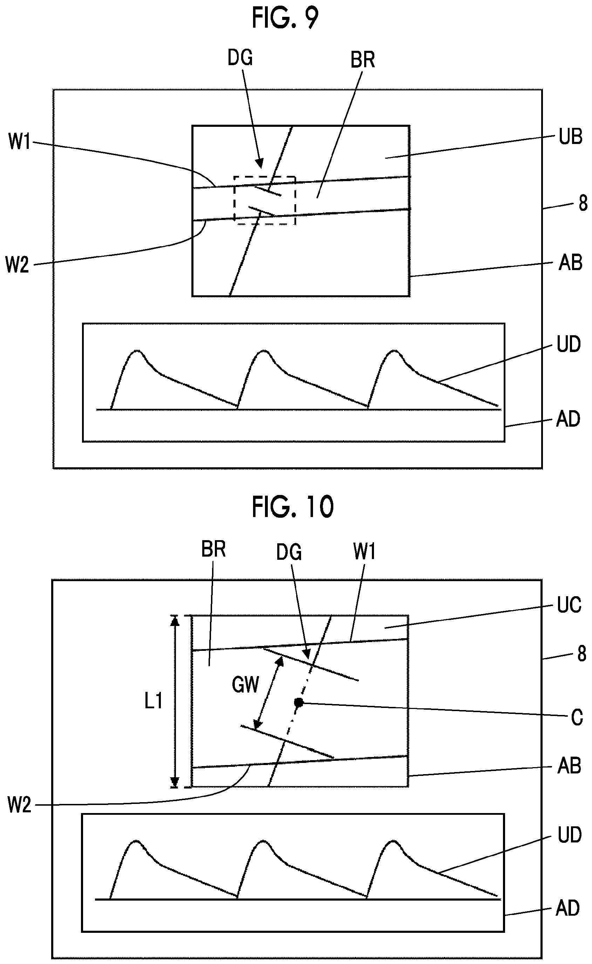

Here, for example, in a case where the user draws the blood vessel region BR in the subject as the measurement target, the user normally draws a wide region including not only the blood vessel region BR but also the peripheral tissues thereof, as illustrated in Fig. 9. In a state where such a B-mode image UB is displayed on the display unit 8, for example, the Doppler gate DG is set on the B-mode image UB, and in a case where both the B-mode image UB and the Doppler waveform image UD are frozen by the user, the image enlargement unit 9 displays an enlarged B-mode image UC in which the blood vessel region BR is enlarged, on the display unit 8, as illustrated in Fig. 10. In this case, the image enlargement unit 9 can enlarge the blood vessel region BR such that the center position C of the set Doppler gate is the center position of the enlarged B-mode image UC, for example.

For the B-mode image UB generated by the B-mode processing unit 5 and the enlarged B-mode image UC in which the blood vessel region BR is enlarged by the image enlargement unit 9, the cross-sectional area calculation unit 12 of the processor 21 calculates a blood vessel diameter DB from the positions of the anterior vascular wall W1 and the posterior vascular wall W2 detected by the vascular wall detection unit 10, and calculates a cross-sectional area of the blood vessel from the blood vessel diameter DB assuming that the blood vessel has a circular cross section.

The average blood flow velocity calculation unit 14 of the processor 21 calculates an average blood flow velocity for one heartbeat period on the basis of the blood flow velocity calculated by the Doppler processing unit 6.

The blood flow rate measurement unit 13 of the processor 21 measures a blood flow rate representing the volume of the blood flowing in the blood vessel per unit time on the basis of the cross-sectional area of the blood vessel calculated by the cross-sectional area calculation unit 12 and the average blood flow velocity calculated by the average blood flow velocity calculation unit 14.

Information on the Doppler gate DG set by the gate setting unit 11 and the blood flow rate measured by the blood flow rate measurement unit 13 is sent to the display unit 8 via the display controller 7 and is displayed on the display unit 8.

The storage unit 17 stores an operation program and the like of the ultrasound diagnostic apparatus 1, and recording media such as a hard disk drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), and a Universal Serial Bus memory (USB memory), a server, or the like can be used.

The processor 21 having the transmission unit 3, the reception unit 4, the B-mode processing unit 5, the Doppler processing unit 6, the display controller 7, the image enlargement unit 9, the vascular wall detection unit 10, the gate setting unit 11, the cross-sectional area calculation unit 12, the blood flow rate measurement unit 13, the average blood flow velocity calculation unit 14, the device controller 15, and the position designation acceptance unit 18 is configured by a central processing unit (CPU) and a control program for causing the CPU to execute various kinds of processing, but the processor 21 may be configured by using a field programmable gate array (FPGA), a digital signal processor (DSP), an application specific integrated circuit (ASIC), a graphics processing unit (GPU), or other integrated circuits (IC) or may be configured by a combination thereof.

In addition, the transmission unit 3, the reception unit 4, the B-mode processing unit 5, the Doppler processing unit 6, the display controller 7, the image enlargement unit 9, the vascular wall detection unit 10, the gate setting unit 11, the cross-sectional area calculation unit 12, the blood flow rate measurement unit 13, the average blood flow velocity calculation unit 14, the device controller 15, and the position designation acceptance unit 18 of the processor 21 can also be configured by being integrated partially or entirely into one CPU or the like.

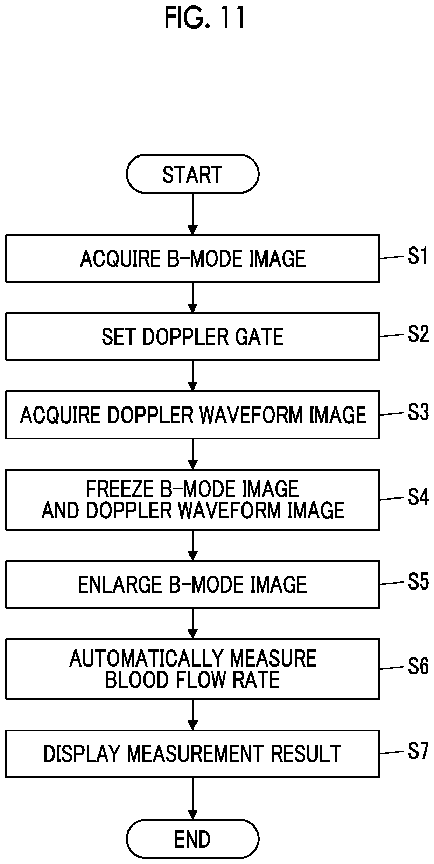

Next, the operation of the ultrasound diagnostic apparatus 1 in the first embodiment will be described in detail using the flowchart illustrated in Fig. 11.

First, in Step S1, the B-mode processing unit 5 sequentially acquires the B-mode images UB in which at least the blood vessel region BR is imaged, and displays the acquired B-mode images UB on the display unit 8. For example, as illustrated in Fig. 9, the display unit 8 has a B-mode image display region AB for displaying the B-mode image UB and a Doppler waveform image display region AD for displaying the Doppler waveform image UD, and the B-mode images UB sequentially acquired by the B-mode processing unit 5 are displayed in the B-mode image display region AB.

Next, in Step S2, the Doppler gate DG is set on the B-mode image UB by the gate setting unit 11. In this case, for example, in a case where the position on the blood vessel region BR in the B-mode image UB is designated by the user through the input unit 16, the anterior vascular wall W1 and the posterior vascular wall W2 are detected by the vascular wall detection unit 10 on the basis of the designated position, and the Doppler gate DG is set by the gate setting unit 11 on the basis of the detected anterior vascular wall W1 and posterior vascular wall W2.

In Step S3, the Doppler waveform images UD are sequentially generated by the Doppler processing unit 6 on the basis of the Doppler gate DG set in Step S2, and the generated Doppler waveform images UD are displayed in the Doppler waveform image display region AD of the display unit 8 as illustrated in Fig. 9.

In this manner, in a state where the B-mode image UB is displayed in the B-mode image display region AB and the Doppler waveform image UD is displayed in the Doppler waveform image display region AD, both the B-mode image UB and the Doppler waveform image UD displayed on the display unit 8 are frozen by the user in Step S4. More specifically, for example, in a case where information on an instruction to freeze both the B-mode image UB and the Doppler waveform image UD is input by the user through the input unit 16 and the input instruction information is transmitted to the display controller 7 via the device controller 15, both the B-mode image UB and the Doppler waveform image UD are frozen under the control of the display controller 7.

In a case where both the B-mode image UB and the Doppler waveform image UD are frozen in this manner, the process proceeds to Step S5, the enlarged B-mode image UC in which the blood vessel region BR including the Doppler gate DG is enlarged is displayed in the B-mode image display region AB of the display unit 8 by the image enlargement unit 9 as illustrated in Fig. 10. The image enlargement unit 9 can enlarge the blood vessel region BR such that the center position C of the Doppler gate DG is the center position of the enlarged B-mode image UC, for example.

Further, the image enlargement unit 9 can enlarge the blood vessel region BR such that a gate width GW of the Doppler gate DG in the enlarged B-mode image UC has a predetermined value, for example. For example, the image enlargement unit 9 can enlarge the blood vessel region BR such that the gate width GW of the Doppler gate DG in the enlarged B-mode image UC has a length obtained by multiplying the size of the B-mode image display region AB, for example, a length L1 in the vertical direction by a certain ratio such as 50% or 90%. The magnification in a case where the image enlargement unit 9 enlarges the blood vessel region BR can be stored in advance by the image enlargement unit 9. Further, the magnification can be set by the user through the input unit 16, for example.

In a case where the enlarged B-mode image UC in which the blood vessel region BR is enlarged in this manner is displayed on the display unit 8, the user can clearly check the blood vessel region BR as the measurement target.

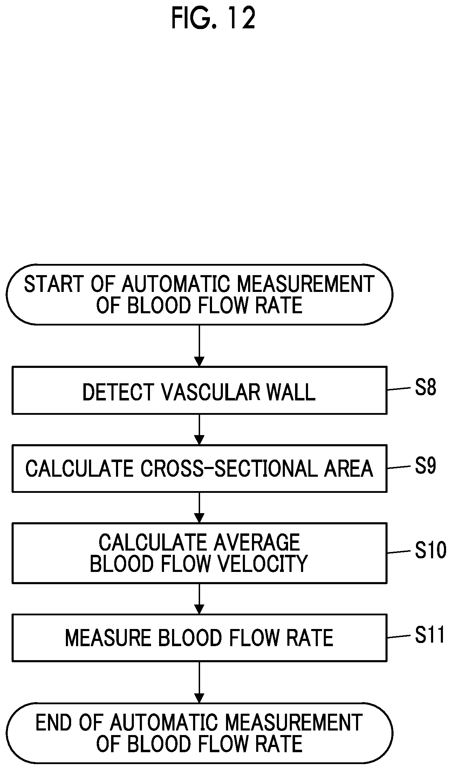

Subsequently, in Step S6, the blood flow rate in the blood vessel region BR is automatically measured. Step S6 will be described using the flowchart illustrated in Fig. 12.

First, in Step S8, the anterior vascular wall W1 and the posterior vascular wall W2 in the enlarged B-mode image UC are detected by the vascular wall detection unit 10. In this case, the vascular wall detection unit 10 can detect the anterior vascular wall W1 and the posterior vascular wall W2 in the enlarged B-mode image UC by using again the designation position SP designated by the user in the B-mode image UB before the enlargement, for example.

In Step S9, by the cross-sectional area calculation unit 12, the blood vessel diameter DB is calculated on the basis of the anterior vascular wall W1 and the posterior vascular wall W2 in the enlarged B-mode image UC detected in Step S8, and the cross-sectional area of the blood vessel is calculated from the calculated blood vessel diameter DB assuming that the blood vessel has a circular cross section.

In Step S10, by the Doppler processing unit 6, the blood flow velocity is calculated on the basis of the Doppler data in the Doppler gate DG disposed on the enlarged B-mode image UC, and the Doppler waveform image UD is newly generated. In this manner, the average blood flow velocity for one heartbeat period is calculated by the average blood flow velocity calculation unit 14 on the basis of the blood flow velocity calculated in this manner.

Finally, in step S11, the blood flow rate representing the volume of the blood flowing in the blood vessel per unit time is measured by the blood flow rate measurement unit 13 on the basis of the cross-sectional area of the blood vessel calculated in step S9 and the average blood flow velocity calculated in Step S10.

Thus, in Step S6, the series of processing of Step S8 to Step S11 is automatically performed on the enlarged B-mode image UC.

Here, for example, in a case where the user draws the blood vessel region BR in the subject as the measurement target on the B-mode image UB, the user draws a wide region including not only the blood vessel region BR but also the peripheral tissues thereof. Thus, in a case where the blood flow rate in the subject is measured using the B-mode image UB on which a wide region is drawn, for example, due to the resolution of the B-mode image UB, an error may occur in the positions of the anterior vascular wall W1 and the posterior vascular wall W2, the disposed position of the Doppler gate DG, and the like, and the measurement accuracy of the blood flow rate may be decreased. For example, in a case where the resolution of the B-mode image UB that has not been enlarged is 0.18 mm/pixel, in a case where the blood vessel diameter DB is 4 mm, an error of 0.18/4, that is, an error of about 4.5% occurs in the blood vessel diameter DB only by the positions of the anterior vascular wall W1 and the posterior vascular wall W2 being shifted by one pixel. This is an error of about 9% in a case of being converted to the blood flow rate. On the other hand, for example, in a case where the B-mode image UB is enlarged 2.5 times, the resolution of the enlarged B-mode image UC is 0.07 mm/pixel, and therefore, for the blood vessel diameter DB of 4 mm, only an error of 0.07/4, that is, an error of about 1.8% occurs in the blood vessel diameter DB by the position of the anterior vascular wall W1 or the posterior vascular wall W2 being shifted by one pixel. This is only an error of about 3.6% in a case of being converted to the blood flow rate.

In the ultrasound diagnostic apparatus 1 of the first embodiment of the invention, since the automatic measurement of the blood flow rate in Step S6 is performed on the enlarged B-mode image UC in which the blood vessel region BR including the Doppler gate DG is enlarged, it is possible to suppress the occurrence of errors and improve the measurement accuracy of the blood flow rate.

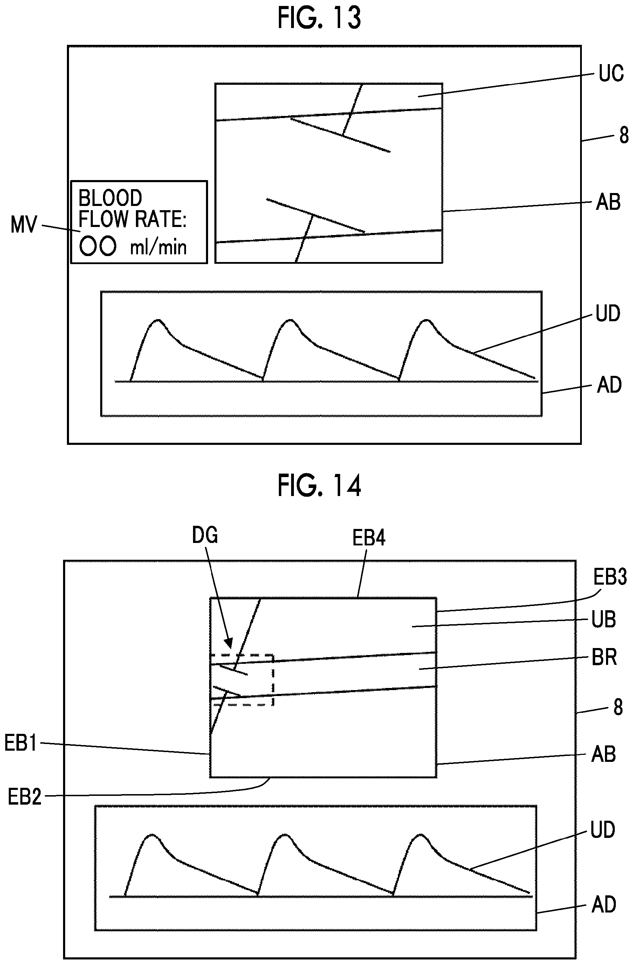

In Step S7, the measurement result of the blood flow rate obtained in Step S6 is displayed on the display unit 8. For example, as illustrated in Fig. 13, a measurement value MV of the blood flow rate is displayed on the display unit 8 together with the enlarged B-mode image UC and the Doppler waveform image UD.

In this manner, in a case where the measurement result of the blood flow rate is displayed on the display unit 8, the operation of the ultrasound diagnostic apparatus 1 is ended.

With the ultrasound diagnostic apparatus 1 according to the first embodiment of the invention, in a case where both the B-mode image UB and the Doppler waveform image UD are frozen by the user, since the enlarged B-mode image UC in which the blood vessel region BR including the Doppler gate DG is enlarged is displayed on the display unit 8 and the measurement of the blood flow rate is performed using the enlarged B-mode image UC by the vascular wall detection unit 10, the cross-sectional area calculation unit 12, the Doppler processing unit 6, the average blood flow velocity calculation unit 14, and the blood flow rate measurement unit 13, it is possible for the user to clearly check the blood vessel region BR on the enlarged B-mode image UC, and it is possible to improve the measurement accuracy of the blood flow rate.

Similar to the method disclosed in JP4749592B, in the first embodiment, the anterior vascular wall W1 and the posterior vascular wall W2 are detected by performing the image analysis on the B-mode image UB, but the method is not limited thereto as long as the anterior vascular wall W1 and the posterior vascular wall W2 can be detected. For example, although not illustrated, a blood vessel gradient line representing a blood vessel gradient is detected by performing the image analysis on the B-mode image UB, and the detection of the anterior vascular wall W1 and the posterior vascular wall W2 can be performed along a gradient perpendicular line perpendicular to the blood vessel gradient line.

Further, in the first embodiment, in a case where the user designates the position on the B-mode image UB through the input unit 16, the anterior vascular wall W1 and the posterior vascular wall W2 in the B-mode image UB are detected by the vascular wall detection unit 10, and the Doppler gate DG is automatically set by the gate setting unit 11 on the basis of the anterior vascular wall W1 and the posterior vascular wall W2, but the Doppler gate DG can be manually set by the user through the input unit 16.

In addition, an example in which the center position C of the Doppler gate DG is used as the reference point in a case where the blood vessel region BR is enlarged, that is, an example in which the blood vessel region BR is enlarged such that the center position C of the Doppler gate DG is the center position of the enlarged B-mode image UC is exemplified, but the reference point in a case where the blood vessel region BR is enlarged is not limited to the center position C of the Doppler gate DG as long as the blood vessel region BR including the Doppler gate DG is enlarged. For example, the position in the blood vessel region BR on the B-mode image UB is designated by the user, both the B-mode image UB and the Doppler waveform image UD are frozen, and thereby the blood vessel region BR can be enlarged by using the designation position SP designated by the user as the reference for the enlargement.

In the first embodiment, a case in which the Doppler waveform image UD is acquired by the Doppler processing unit 6 with the setting of the Doppler gate DG on the B-mode image UB as a trigger is exemplified, but the trigger for acquiring the Doppler waveform image UD is not limited thereto.

For example, the Doppler waveform image UD may be acquired by the Doppler processing unit 6 with the setting of the Doppler gate DG on the B-mode image UB and the freezing of the B-mode image UB by the user through the input unit 16 as the trigger. Thereby, only the B-mode image UB is frozen and the Doppler waveform image UD is displayed on the display unit 8, but, for example, in a case where the Doppler waveform image UD is frozen by the user through the input unit 16 so that both the B-mode image UB and the Doppler waveform image UD are frozen, the enlarged B-mode image UC in which the blood vessel region BR including the Doppler gate DG is enlarged is displayed on the display unit 8, by the image enlargement unit 9.

For example, the Doppler waveform image UD may be acquired by the Doppler processing unit 6 with the freezing of the B-mode image UB in a state where the B-mode image UB is acquired by the B-mode processing unit 5 and is displayed on the display unit 8 and the setting of the Doppler gate DG on the frozen B-mode image UB as the trigger. Also in this case, similar to the case in which the Doppler waveform image UD is acquired with the freezing of the B-mode image UB as the trigger, for example, in a case where the Doppler waveform image UD is frozen by the user through the input unit 16 so that both the B-mode image UB and the Doppler waveform image UD are frozen, the enlarged B-mode image UC in which the blood vessel region BR including the Doppler gate DG is enlarged is displayed on the display unit 8, by the image enlargement unit 9.

Further, the B-mode image UB and the Doppler waveform image UD can be displayed on the display unit 8 by switching between a state in which only the B-mode image UB is frozen and a state in which only the Doppler waveform image UD is frozen, of the B-mode image UB and the Doppler waveform image UD. For example, although not illustrated, a display switching button for switching between the state in which only the B-mode image UB is frozen and the state in which only the Doppler waveform image UD is frozen on the display unit 8 is displayed on the display unit 8, and the display switching button is pushed by the user through the input unit 16 so that the state in which only the B-mode image UB is frozen and the state in which only the Doppler waveform image UD is frozen are switch to be displayed on the display unit 8. In this case, for example, in a case where the user determines that the measurement of the blood flow rate cannot be appropriately performed as in a case where the B-mode image UB or the Doppler waveform image UD frozen on the display unit 8 is unclear or the like, the user pushes the display switching button, and the newly acquired B-mode image UB or Doppler waveform image UD is frozen so that the B-mode image UB or the Doppler waveform image UD to be used for the measurement of the blood flow rate can be newly set again.

The frozen state of the B-mode image UB and the Doppler waveform image UD may be individually released by the user through the input unit 16.

Further, an example in which the blood vessel region BR is enlarged by the image enlargement unit 9 such that the gate width GW of the Doppler gate DG disposed in the blood vessel region BR has a predetermined value is exemplified, but, for example, the blood vessel region BR can be enlarged such that the distance between the anterior vascular wall W1 and the posterior vascular wall W2 in the enlarged B-mode image UC has a predetermined value. For example, the image enlargement unit 9 can enlarge the blood vessel region BR such that the distance between the anterior vascular wall W1 and the posterior vascular wall W2 in the enlarged B-mode image UC has a length obtained by multiplying the size of the B-mode image display region AB, for example, the length L1 in the vertical direction by a certain ratio such as 50% or 90%. The magnification in a case where the image enlargement unit 9 enlarges the blood vessel region BR can be stored in advance by the image enlargement unit 9. Further, the magnification can be set by the user through the input unit 16, for example.

In a case where the magnification of the blood vessel region BR is set by the user, the image enlargement unit 9 holds the magnification of the blood vessel region BR set by the user, and can display the enlarged B-mode image UC in which the blood vessel region BR is enlarged using the held magnification, on the display unit 8 in a case of measuring the blood flow rate from the next time onward. Thereby, it is possible to save the labor for resetting the magnification of the blood vessel region BR each time the user performs the measurement of the blood flow rate of the subject.

In the first embodiment, a case where the blood flow rate in the blood vessel is automatically measured is exemplified, but, for example, the processing in Step S5 to Step S8, that is, the detection of the vascular wall, the calculation of the cross-sectional area of the blood vessel, the calculation of the average blood flow velocity, and the measurement of the blood flow rate may be performed with an instruction manually input by the user through the input unit 16 as the trigger. For example, a measurement caliper for designating two points for measuring the blood vessel diameter is manually disposed on the anterior vascular wall W1 and the posterior vascular wall W2 by the user through the input unit 16, and the cross-sectional area of the blood vessel can be calculated by the cross-sectional area calculation unit 12 on the basis of the disposed measurement caliper.

### Second Embodiment

In the first embodiment, the example in which the blood vessel region BR is enlarged by the image enlargement unit 9 such that the center position C of the Doppler gate DG is the center position of the enlarged B-mode image UC has been exemplified, but the method of enlarging the blood vessel region BR is not limited thereto.

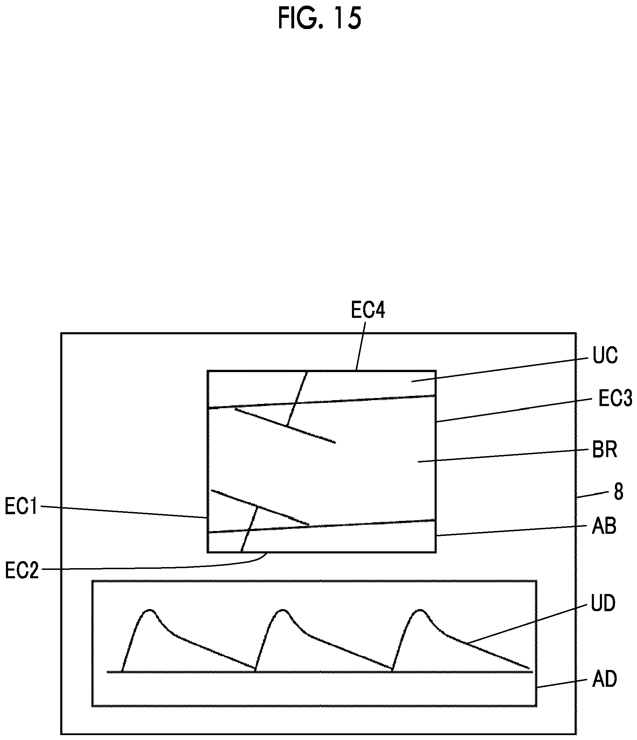

For example, in a case where the blood vessel region BR to be enlarged is close to a left end portion EB 1 of the B-mode image UB since the Doppler gate DG is positioned near the left end portion EB1 of the B-mode image UB as illustrated in Fig. 14, the image enlargement unit 9 can enlarge the blood vessel region BR such that the left end portion EB1 of the B-mode image UB is a left end portion EC1 of the enlarged B-mode image UC as illustrated in Fig. 15.

The blood vessel region BR to be enlarged is close to the left end portion EB1 of the B-mode image UB in the example illustrated in Fig. 14, but in a case where the blood vessel region BR to be enlarged is close to a lower end portion EB2 of the B-mode image UB, the blood vessel region BR is enlarged such that the lower end portion EB2 of the B-mode image UB is a lower end portion EC2 of the enlarged B-mode image UC, in a case where the blood vessel region BR to be enlarged is close to a right end portion EB3 of the B-mode image UB, the blood vessel region BR is enlarged such that the right end portion EB3 of the B-mode image UB is a right end portion EC3 of the enlarged B-mode image UC, and in a case where the blood vessel region BR to be enlarged is close to an upper end portion EB4 of the B-mode image UB, the blood vessel region BR is enlarged such that the upper end portion EB4 of the B-mode image UB is an upper end portion EC4 of the enlarged B-mode image UC. Further, in a case where the blood vessel region BR to be enlarged is close to two adjacent end portions of the B-mode image UB, for example, both the left end portion EB1 and the lower end portion EB2 of the B-mode image UB, the blood vessel region BR is enlarged such that the left end portion EB 1 and the lower end portion EB2 of the B-mode image UB are the left end portion EC1 and the lower end portion EC2 of the enlarged B-mode image UC.

Thus, in the second embodiment, in a case where the blood vessel region BR to be enlarged is close to the end portion of the B-mode image UB, the blood vessel region BR including the Doppler gate DG is enlarged by the image enlargement unit 9 such that the end portion of the B-mode image UB is the end portion of the enlarged B-mode image UC, and therefore, even in a case where the Doppler gate DG is positioned near the end portion of the B-mode image UB, the enlarged B-mode image UC including the Doppler gate DG is displayed in the entire B-mode image display region AB, and the user can clearly check the blood vessel region BR.

### Third Embodiment

In the first embodiment and the second embodiment, the image enlargement unit 9 holds only one magnification, and enlarges the blood vessel region BR on the B-mode image UB using the magnification, but the image enlargement unit 9 may hold a plurality of predetermined magnifications which are different from each other. In the third embodiment, it is assumed that the image enlargement unit 9 holds a plurality of magnifications such as 1.5 times, 2.0 times, 2.5 times, 3.0 times, 3.5 times, and 4.0 times as the magnification of the B-mode image UB, for example.

First, in Step S1, by the B-mode processing unit 5, the B-mode images UB in which at least the blood vessel region BR is imaged are sequentially acquired, and the acquired B-mode images UB are displayed on the display unit 8.

Next, in Step S2, the Doppler gate DG is set on the B-mode image UB by the gate setting unit 11.

In subsequent Step S3, the Doppler waveform images UD are sequentially generated by the Doppler processing unit 6 on the basis of the Doppler gate DG set in Step S2, and the generated Doppler waveform images UD are displayed in the Doppler waveform image display region AD of the display unit 8.

In Step S4, both the B-mode image UB displayed in the B-mode image display region AB and the Doppler waveform image UD on the display unit 8 are frozen by the user through the input unit 16.

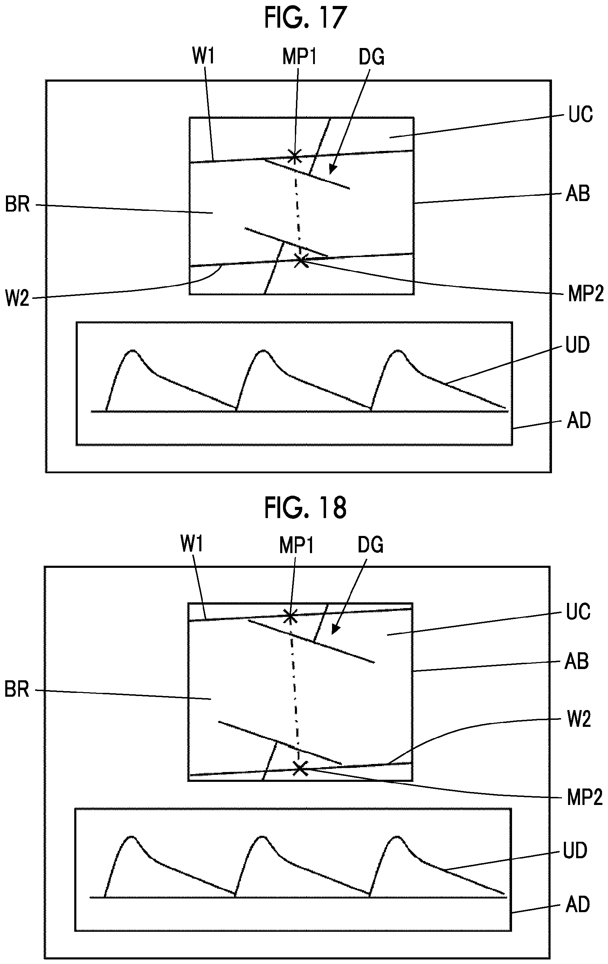

In a case where both the B-mode image UB and the Doppler waveform image UD are frozen in this manner, in Step S5, the enlarged B-mode image UC in which the blood vessel region BR including the Doppler gate DG is enlarged is displayed in the B-mode image display region AB of the display unit 8 by the image enlargement unit 9 as illustrated in Fig. 17. In this case, the image enlargement unit 9 displays the enlarged B-mode image UC in which the blood vessel region BR is enlarged using one magnification, for example, the lowest magnification among the plurality of magnifications held by the image enlargement unit 9, on the display unit 8.

Subsequently, in Step S6, the blood flow rate in the blood vessel is automatically measured. In a case where the measurement of the blood flow rate in Step S6 is completed, the process proceeds to Step S12.

In Step S12, it is determined whether the anterior vascular wall W1 and the posterior vascular wall W2 are within the B-mode image display region AB even in a case where the enlarged B-mode image UC displayed on the display unit 8 in Step S2 is enlarged at a magnification that is one step larger than the magnification used in Step S5. Here, in a case where it is determined that the anterior vascular wall W1 and the posterior vascular wall W2 are within the B-mode image display region AB, the process proceeds to Step S13.

In Step S13, the image enlargement unit 9 displays, on the display unit 8, the enlarged B-mode image UC in which the B-mode image UB is enlarged again at a magnification that is one step larger than the magnification used in Step S5. In a case where the enlarged B-mode image UC is displayed on the display unit 8 in this manner, the process returns to Step S6.

In Step S6, the blood flow rate is automatically measured by using the enlarged B-mode image UC displayed on the display unit 8 in Step S13.

In subsequent Step S12, it is determined whether the anterior vascular wall W1 and the posterior vascular wall W2 are within the B-mode image display region AB even in a case where the B-mode image UB is enlarged at a magnification that is one step larger than the magnification used in Step S13. In a case where it is determined that the anterior vascular wall W1 and the posterior vascular wall W2 are within the B-mode image display region AB even in a case where the B-mode image UB is enlarged at a magnification that is one step larger than the magnification used in Step S13, the process proceeds to Step S13.

In Step S13, the image enlargement unit 9 displays, on the display unit 8, the enlarged B-mode image UC in which the B-mode image UB is enlarged again at a magnification that is one step larger than the magnification used in previous Step S13.

In a case where the enlarged B-mode image UC is displayed on the display unit 8 in Step S13, the process proceeds to Step S6, and the blood flow rate is automatically measured by using the enlarged B-mode image UC displayed on the display unit 8 in Step S13. In this manner, in a case the B-mode image UB is further enlarged by one step in Step S13, the processing of Step S6, Step S12, and Step S13 is repeated until it is determined that the anterior vascular wall W1 and the posterior vascular wall W2 are not within the B-mode image display region AB.

As a result of repeating the processing of Step S6, Step S12, and Step S13, for example, as illustrated in Fig 18, the enlarged B-mode image UC in which the B-mode image UB is enlarged by the maximum magnification where the anterior vascular wall W1 and the posterior vascular wall W2 are within the B-mode image display region AB is displayed on the display unit 8, and in a case where it is determined in Step S12 that the anterior vascular wall W1 and the posterior vascular wall W2 are not within the B-mode image display region AB in a case where the B-mode image UB is further enlarged by one step, the process proceeds to Step S7. Even in a case where the enlarged B-mode image UC in which the blood vessel region BR is enlarged is used in this manner, for example, two measurement points MP1 and MP2 for measuring the blood vessel diameter are respectively disposed on the anterior vascular wall W1 and the posterior vascular wall W2 so that the blood vessel diameter is calculated, the cross-sectional area of the blood vessel is calculated from the calculated blood vessel diameter, the average blood flow velocity is calculated on the basis of the Doppler data in the Doppler gate DG, and the blood flow rate is calculated on the basis of the calculated cross-sectional area of the blood vessel and the calculated average blood flow velocity.

In Step S7, the blood flow rate measured in Step S3 is displayed on the display unit 8, the operation of the ultrasound diagnostic apparatus 1 according to the third embodiment is ended.

With the ultrasound diagnostic apparatus 1 of the third embodiment of the invention, the image enlargement unit 9 holds a plurality of predetermined magnifications which are different from each other, and the blood vessel region BR is enlarged by the maximum magnification where the anterior vascular wall W1 and the posterior vascular wall W2 are included in the B-mode image display region AB, among the plurality of predetermined magnifications, so that the enlarged B-mode image UC in which the blood vessel region BR is enlarged using the maximum magnification within a range where the blood flow rate can be measured can be displayed on the display unit 8. Therefore, it is possible for the user to clearly check the blood vessel region BR, and it is possible to improve the measurement accuracy of the blood flow rate.

The example in which the blood flow rate is automatically measured using the enlarged B-mode image UC each time the B-mode image UB is enlarged by the image enlargement unit 9 has been described, but the vascular wall detection unit 10, the cross-sectional area calculation unit 12, the Doppler processing unit 6, the average blood flow velocity calculation unit 14, and the blood flow rate measurement unit 13 can also automatically measure the blood flow rate using the enlarged B-mode image UC for the first time after the maximum magnification where the anterior vascular wall W1 and the posterior vascular wall W2 are included in the B-mode image display region AB is decided.

### Fourth Embodiment

In the first embodiment to the third embodiment, the display unit 8 has the B-mode image display region AB and the Doppler waveform image display region AD which respectively have predetermined sizes, but the size ratio of these regions may be changed according to the enlargement of the enlarged B-mode image UC, for example.

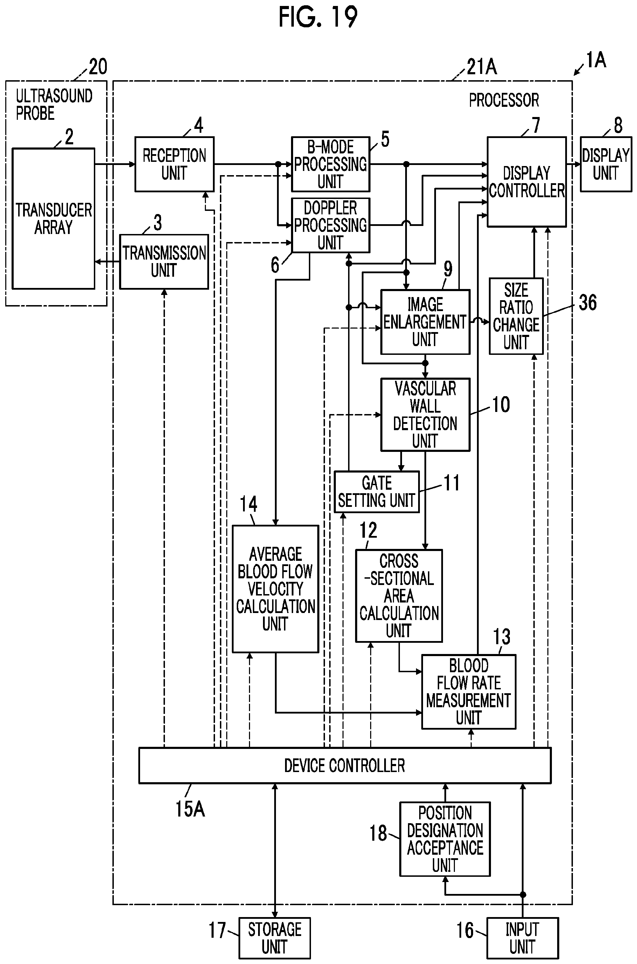

Fig. 19 illustrates a configuration of an ultrasound diagnostic apparatus 1A according to a fourth embodiment of the invention. The ultrasound diagnostic apparatus 1A is obtained by comprising a device controller 15A instead of the device controller 15 and adding a size ratio change unit 36 to the ultrasound diagnostic apparatus 1 of the first embodiment illustrated in Fig. 1. In the ultrasound diagnostic apparatus 1A, the size ratio change unit 36 is connected to the image enlargement unit 9, and the display controller 7 is connected to the size ratio change unit 36. Further, the device controller 15A is connected to the size ratio change unit 36, and the transmission unit 3, the reception unit 4, the B-mode processing unit 5, the Doppler processing unit 6, the display controller 7, the image enlargement unit 9, the vascular wall detection unit 10, the gate setting unit 11, the cross-sectional area calculation unit 12, the blood flow rate measurement unit 13, the average blood flow velocity calculation unit 14, the device controller 15A, the position designation acceptance unit 18, and the size ratio change unit 36 constitute a processor 21A.

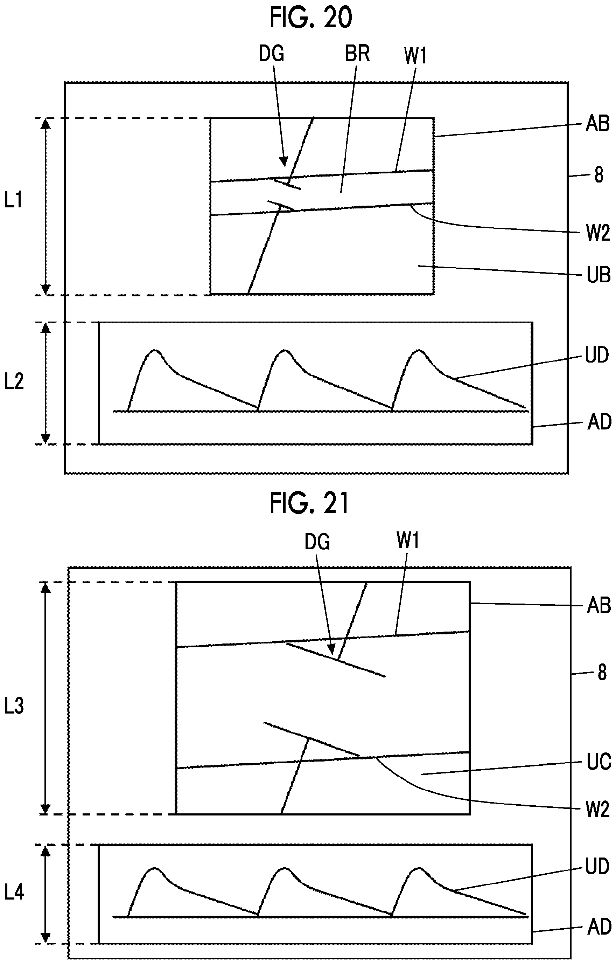

The size ratio change unit 36 of the processor 21A changes a size ratio of the B-mode image display region AB and the Doppler waveform image display region AD via the display controller 7 on the basis of the size of the enlarged B-mode image UC generated by the image enlargement unit 9. For example, in a case where the any one of the anterior vascular wall W1, the posterior vascular wall W2, or the Doppler gate DG in the enlarged B-mode image UC in which the blood vessel region BR of the B-mode image UB illustrated in Fig. 20 is enlarged is not within the B-mode image display region AB, the size ratio change unit 36 can increase the size of the B-mode image display region AB and reduce the size of the Doppler waveform image display region AD as illustrated in Fig. 21 in order to cause the anterior vascular wall W1, the posterior vascular wall W2, and the Doppler gate DG to be within the B-mode image display region AB.

In this case, the size ratio change unit 36 holds a predetermined size ratio of the B-mode image display region AB and the Doppler waveform image display region AD in advance, and can change the size ratio of the B-mode image display region AB and the Doppler waveform image display region AD to the size ratio held in advance.

Here, as the size ratio of the B-mode image display region AB and the Doppler waveform image display region AD, as illustrated in Fig. 20, the ratio of the length L1 of the B-mode image display region AB in the vertical direction and a length L2 of the Doppler waveform image display region AD in the vertical direction can be used. In the example illustrated in Fig. 21, by changing the size ratio of the B-mode image display region AB and the Doppler waveform image display region AD, the length of the B-mode image display region AB in the vertical direction is changed from L1 to L3, and the length of the Doppler waveform image display region AD in the vertical direction is changed from L2 to L4.

In the ultrasound diagnostic apparatus 1A according to the fourth embodiment, since the size ratio of the B-mode image display region AB and the Doppler waveform image display region AD is changed via the display controller 7 on the basis of the size of the enlarged B-mode image UC in this manner so that the B-mode image display region AB can be displayed larger, it is possible for the user to further clearly check the blood vessel region BR on the B-mode image, and it is possible to improve the measurement accuracy of the blood flow rate.

The size ratio change unit 36 holds the size ratio of the B-mode image display region AB and the Doppler waveform image display region AD in advance, but the size ratio can also be set by the user. For example, a value of the size ratio can be input by the user through the input unit 16, and the input size ratio can be held in the size ratio change unit 36.

Further, as the size ratio of the B-mode image display region AB and the Doppler waveform image display region AD, the size ratio change unit 36 uses the ratio of the length L1 of the B-mode image display region AB in the vertical direction and a length L2 of the Doppler waveform image display region AD in the vertical direction, but the size ratio change unit 36 can use an area ratio of the B-mode image display region AB and the Doppler waveform image display region AD, for example.

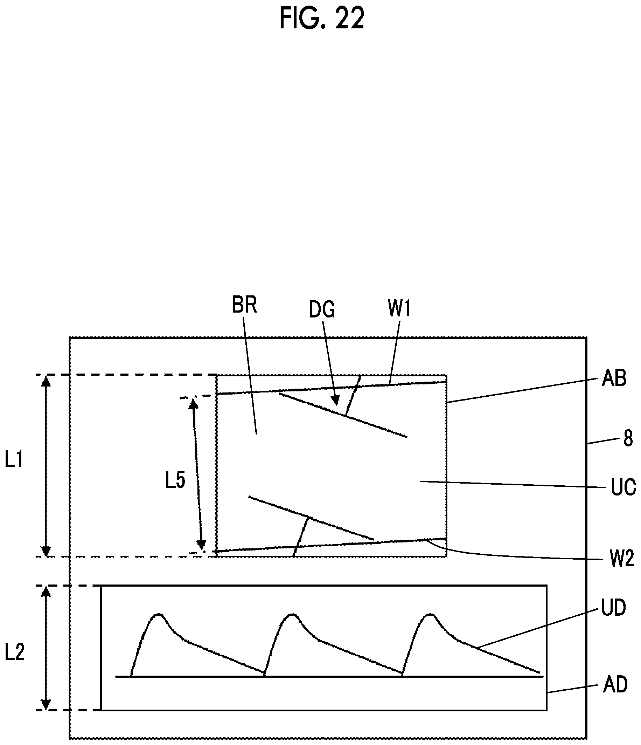

The size ratio change unit 36 changes the size ratio of the B-mode image display region AB and the Doppler waveform image display region AD in a case where any one of the anterior vascular wall W1, the posterior vascular wall W2, or the Doppler gate DG in the enlarged B-mode image UC is not within the B-mode image display region AB, but the trigger for the size ratio change unit 36 to change the size ratio is not limited thereto. For example, as illustrated in Fig. 22, in a case where the magnification of the enlarged B-mode image UC is large so that it is difficult for the user to grasp the positional relationship between the region outside the blood vessel region BR and the blood vessel region BR, the size ratio of the B-mode image display region AB and the Doppler waveform image display region AD is changed so that the B-mode image display region AB can be displayed even larger on the display unit 8. Specifically, for example, in a case where a ratio of a length L5 between the anterior vascular wall W1 and the posterior vascular wall W2 to the length L1 of the B-mode image display region AB in the vertical direction is equal to or greater than a predetermined value such as 80% or 90%, the size ratio change unit 36 can change the size ratio of the B-mode image display region AB and the Doppler waveform image display region AD.

### Fifth Embodiment

The ultrasound diagnostic apparatus 1 of the first embodiment to the third embodiment has the configuration in which the display unit 8, the input unit 16, and the ultrasound probe 20 are directly connected to the processor 21, but, for example, the display unit 8, the input unit 16, the ultrasound probe 20, and the processor 21 can be indirectly connected to each other via the network.

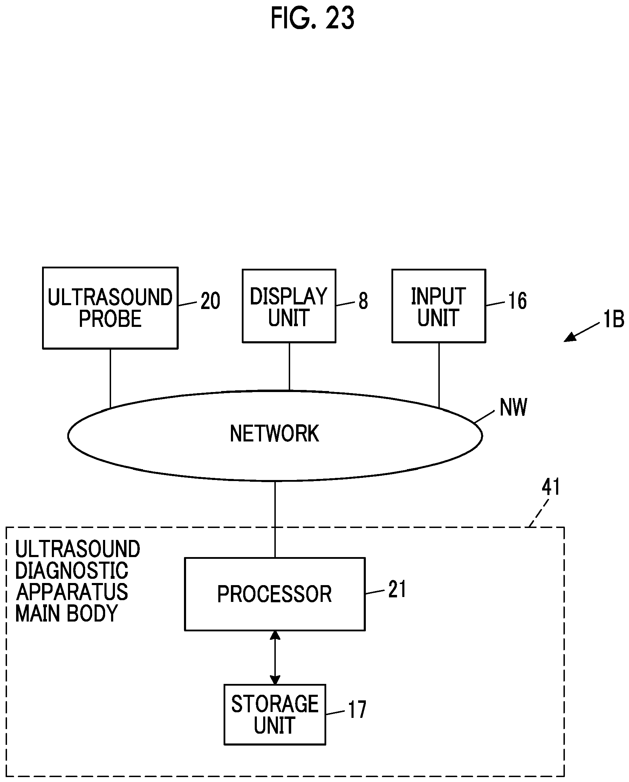

As illustrated in Fig. 23, in an ultrasound diagnostic apparatus 1B in a fifth embodiment, the display unit 8, the input unit 16, and the ultrasound probe 20 are connected to an ultrasound diagnostic apparatus main body 41 via a network NW. The ultrasound diagnostic apparatus main body 41 is obtained by excluding the display unit 8, the input unit 16, and the ultrasound probe 20 in the ultrasound diagnostic apparatus 1 of the first embodiment illustrated in Fig. 1, and is constituted by the processor 21 and the storage unit 17.

Even in a case where the ultrasound diagnostic apparatus 1B has such a configuration, similar to the ultrasound diagnostic apparatus 1 of the first embodiment, in a case where both the B-mode image UB and the Doppler waveform image UD are frozen by the user, the enlarged B-mode image UC in which the blood vessel region BR including the Doppler gate DG is enlarged is displayed on the display unit 8, and the blood flow rate is measured using the enlarged B-mode image UC. Therefore, with the ultrasound diagnostic apparatus 1B, it is possible for the user to clearly check the blood vessel region BR on the enlarged B-mode image UC, and it is possible to improve the measurement accuracy of the blood flow rate.

Further, since the display unit 8, the input unit 16, and the ultrasound probe 20 are connected to the ultrasound diagnostic apparatus main body 41 via the network NW, the ultrasound diagnostic apparatus body 41 can be used as a so-called remote server. Thereby, for example, since the user can perform the diagnosis of the subject by preparing the display unit 8, the input unit 16, and the ultrasound probe 20 at the user's hand, it is possible to improve the convenience in a case of the ultrasound diagnosis.

Further, in a case where a portable thin computer, for example, a so-called tablet, is used as the display unit 8 and the input unit 16, it is possible for the user to more easily perform the ultrasound diagnosis of the subject, and it is possible to further improve the convenience in a case of the ultrasound diagnosis.

The display unit 8, the input unit 16, and the ultrasound probe 20 are connected to the ultrasound diagnostic apparatus main body 41 via the network NW, but in this case, the display unit 8, the input unit 16, and the ultrasound probe 20 may be connected to the network NW in a wired manner or in a wireless manner.

Further, it is described that the form of the fifth embodiment is applied to the first embodiment, but the form of the fifth embodiment can be similarly applied to the second embodiment to the fourth embodiment.

### Explanation of References

1, 1A, 1B: ultrasound diagnostic apparatus
2: transducer array
3: transmission unit
4: reception unit
5: B-mode processing unit
6: Doppler processing unit
7: display controller
8: display unit
9: image enlargement unit
10: vascular wall detection unit
11: gate setting unit
12: cross-sectional area calculation unit
13: blood flow rate measurement unit
14: average blood flow velocity calculation unit
15, 15A: device controller
16: input unit
17: storage unit
18: position designation acceptance unit
20: ultrasound probe
21, 21A: processor
22: amplification unit
23: AD conversion unit
24: beam former
25: signal processing unit
26: DSC
27: image processing unit
28: quadrature detection unit
29: high-pass filter
30: fast Fourier transformer
31: Doppler waveform image generation unit
32: data memory
33: blood vessel region detection unit
34: closed section setting unit
35: closed section search unit
36: size ratio change unit
41: ultrasound diagnostic apparatus main body
AB: B-mode image display region
A1: cursor steering angle
AD: Doppler waveform image display region
BR: blood vessel region
C: center position
DB: blood vessel diameter
DG: Doppler gate
EB1, EC1: left end portion
EB2, EC2: lower end portion
EB3, EC3: right end portion
EB4, EC4: upper end portion
GW: gate width
L1, L2, L3, L4, L5: length
MV: measurement value
MP1, MP2: measurement point
NW: network
R: closed section
SL: scan line
SP: designation position
SV: vertical line
UB: B-mode image
UC: enlarged B-mode image
UD: Doppler waveform image
W1: anterior vascular wall
W2: posterior vascular wall
X, Y: direction

## Claims

1. An ultrasound diagnostic apparatus comprising:
a gate setting unit that sets a Doppler gate in a blood vessel region by performing an image analysis on a B-mode image in which at least the blood vessel region is imaged;
a Doppler processing unit that generates a Doppler waveform image on the basis of Doppler data in the Doppler gate;
a display unit that displays the B-mode image and the Doppler waveform image; and
an image enlargement unit that, in a case where both the B-mode image and the Doppler waveform image are frozen by a user, displays an enlarged B-mode image in which the blood vessel region including the Doppler gate is enlarged, on the display unit; and
wherein the Doppler processing unit calculates a blood flow velocity on the basis of the Doppler data in the Doppler gate, and
the ultrasound diagnostic apparatus further comprises
a vascular wall detection unit that detects an anterior vascular wall and a posterior vascular wall by performing the image analysis on the enlarged B-mode image;
a cross-sectional area calculation unit that calculates a cross-sectional area of a blood vessel on the basis of the anterior vascular wall and the posterior vascular wall detected by the vascular wall detection unit; and
a blood flow rate measurement unit that measures a blood flow rate on the basis of the cross-sectional area of the blood vessel calculated by the cross-sectional area calculation unit and the blood flow velocity calculated by the Doppler processing unit.

2. The ultrasound diagnostic apparatus according to claim 1,
wherein the image enlargement unit displays the blood flow rate measured by the blood flow rate measurement unit together with the enlarged B-mode image on the display unit.

3. The ultrasound diagnostic apparatus according to any one of claims 1 or 2,
wherein the image enlargement unit enlarges the blood vessel region such that a center position of the Doppler gate is a center position of the enlarged B-mode image.

4. The ultrasound diagnostic apparatus according to any one of claims 1 and 2
wherein in a case where the blood vessel region to be enlarged is close to an end portion of the B-mode image, the image enlargement unit enlarges the blood vessel region such that the end portion of the B-mode image is an end portion of the enlarged B-mode image.

5. The ultrasound diagnostic apparatus according to any one of claims 1 to 4,
wherein the image enlargement unit enlarges the blood vessel region such that a gate width of the Doppler gate in the enlarged B-mode image has a predetermined value.

6. The ultrasound diagnostic apparatus according to claim 1,
wherein the image enlargement unit enlarges the blood vessel region such that a distance between the anterior vascular wall and the posterior vascular wall in the enlarged B-mode image has a predetermined value.

7. The ultrasound diagnostic apparatus according to claim 1,
wherein the display unit has a B-mode image display region for displaying the B-mode image, and
the image enlargement unit holds a plurality of predetermined magnifications which are different from each other, and enlarges the blood vessel region by the maximum magnification where the anterior vascular wall and the posterior vascular wall are included in the B-mode image display region, among the plurality of predetermined magnifications.

8. The ultrasound diagnostic apparatus according to claim 1,
wherein the display unit has a B-mode image display region for displaying the B-mode image and a Doppler waveform image display region for displaying the Doppler waveform image, and
the ultrasound diagnostic apparatus further comprises a size ratio change unit that changes a size ratio of the B-mode image display region and the Doppler waveform image display region on the basis of a size of the enlarged B-mode image.

9. The ultrasound diagnostic apparatus according to claim 1,
wherein in a case where both the B-mode image and the Doppler waveform image are frozen by the user, the blood flow rate is automatically measured by the blood flow rate measurement unit.

10. A control method of an ultrasound diagnostic apparatus, the control method comprising:
setting a Doppler gate in a blood vessel region by performing an image analysis on a B-mode image in which at least the blood vessel region is imaged;
calculating a blood flow velocity on the basis of the Doppler data in the Doppler gate
generating a Doppler waveform image on the basis of Doppler data in the Doppler gate;
displaying the B-mode image and the Doppler waveform image on a display unit; and
displaying an enlarged B-mode image in which the blood vessel region including the Doppler gate is enlarged, on the display unit in a case where both the B-mode image and the Doppler waveform image are frozen by a user;
detecting an anterior vascular wall and a posterior vascular wall by performing image analysis on the enlarged B-mode image;
calculating a cross-sectional area of a blood vessel on the basis of the detected anterior vascular wall and posterior vascular wall; and
measuring a blood flow rate on the basis of the calculated cross-sectional area of the blood vessel and the calculated blood flow velocity.

## Patentansprüche

1. Ultraschalldiagnoseeinrichtung, umfassend:
eine Gatter-Einstelleinheit, die ein Doppler-Gatter in einem Blutgefäßbereich einstellt, indem sie eine Bildanalyse an einem B-Modus-Bild durchführt, in dem mindestens der Blutgefäßbereich abgebildet ist;
eine Doppler-Verarbeitungseinheit, die auf der Grundlage von Doppler-Daten im Doppler-Gatter ein Doppler-Wellenformbild erzeugt;
eine Anzeigeeinheit, die das B-Modus-Bild und das Doppler-Wellenformbild anzeigt; und
eine Bildvergrößerungseinheit, die in einem Fall, in dem sowohl das B-Modus-Bild als auch das Doppler-Wellenformbild von einem Benutzer eingefroren werden, ein vergrößertes B-Modus-Bild, in dem der Blutgefäßbereich einschließlich des Doppler-Gatters vergrößert ist, auf der Anzeigeeinheit anzeigt; und
wobei die Doppler-Verarbeitungseinheit eine Blutflussgeschwindigkeit auf der Grundlage der Doppler-Daten im Doppler-Gatter berechnet, und
die Ultraschalldiagnoseeinrichtung weiter umfasst
eine Gefäßwanderkennungseinheit, die eine vordere Gefäßwand und eine hintere Gefäßwand erkennt, indem sie die Bildanalyse am vergrößerten B-Modus-Bild durchführt;
eine Querschnittsflächenberechnungseinheit, die eine Querschnittsfläche eines Blutgefäßes auf der Grundlage der von der Gefäßwanderkennungseinheit erkannten vorderen Gefäßwand und hinteren Gefäßwand berechnet; und
eine Blutflussratenmesseinheit, die eine Blutflussrate auf der Grundlage der von der Querschnittsflächenberechnungseinheit berechneten Querschnittsfläche des Blutgefäßes und der von der Doppler-Verarbeitungseinheit berechneten Blutflussgeschwindigkeit misst.

2. Ultraschalldiagnoseeinrichtung nach Anspruch 1,
wobei die Bildvergrößerungseinheit die von der Blutflussratenmesseinheit gemessene Blutflussrate zusammen mit dem vergrößerten B-Modus-Bild auf der Anzeigeeinheit anzeigt.

3. Ultraschalldiagnoseeinrichtung nach einem der Ansprüche 1 oder 2,
wobei die Bildvergrößerungseinheit den Blutgefäßbereich derart vergrößert, dass eine Mittenposition des Doppler-Gatters eine Mittenposition des vergrößerten B-Modus-Bildes ist.

4. Ultraschalldiagnoseeinrichtung nach einem der Ansprüche 1 und 2,
wobei in einem Fall, in dem der zu vergrößernde Blutgefäßbereich nahe an einem Endabschnitt des B-Modus-Bildes liegt, die Bildvergrößerungseinheit den Blutgefäßbereich derart vergrößert, dass der Endabschnitt des B-Modus-Bildes ein Endabschnitt des vergrößerten B-Modus-Bildes ist.

5. Ultraschalldiagnoseeinrichtung nach einem der Ansprüche 1 bis 4,
wobei die Bildvergrößerungseinheit den Blutgefäßbereich derart vergrößert, dass eine Gatter-Breite des Doppler-Gatters im vergrößerten B-Modus-Bild einen vorbestimmten Wert aufweist.

6. Ultraschalldiagnoseeinrichtung nach Anspruch 1,
wobei die Bildvergrößerungseinheit den Blutgefäßbereich derart vergrößert, dass ein Abstand zwischen der vorderen Gefäßwand und der hinteren Gefäßwand im vergrößerten B-Modus-Bild einen vorbestimmten Wert aufweist.

7. Ultraschalldiagnoseeinrichtung nach Anspruch 1,
wobei die Anzeigeeinheit einen B-Modus-Bildanzeigebereich zum Anzeigen des B-Modus-Bildes aufweist und
die Bildvergrößerungseinheit eine Vielzahl von vorbestimmten Vergrößerungen hält, die sich voneinander unterscheiden, und den Blutgefäßbereich um die maximale Vergrößerung aus der Vielzahl von vorbestimmten Vergrößerungen vergrößert, bei der die vordere Gefäßwand und die hintere Gefäßwand im B-Modus-Bildanzeigebereich eingeschlossen sind.

8. Ultraschalldiagnoseeinrichtung nach Anspruch 1,
wobei die Anzeigeeinheit einen B-Modus-Bildanzeigebereich zum Anzeigen des B-Modus-Bildes und einen Doppler-Wellenform-Bildanzeigebereich zum Anzeigen des Doppler-Wellenformbildes aufweist und
die Ultraschalldiagnoseeinrichtung weiter eine Größenverhältnis-Änderungseinheit umfasst, die ein Größenverhältnis des B-Modus-Bildanzeigebereichs und des Doppler-Wellenform-Bildanzeigebereichs auf der Grundlage einer Größe des vergrößerten B-Modus-Bildes ändert.

9. Ultraschalldiagnoseeinrichtung nach Anspruch 1,
wobei in einem Fall, in dem sowohl das B-Modus-Bild als auch das Doppler-Wellenformbild vom Benutzer eingefroren werden, die Blutflussrate automatisch von der Blutflussratenmesseinheit gemessen wird.

10. Steuerungsverfahren für eine Ultraschalldiagnoseeinrichtung, wobei das Steuerungsverfahren umfasst:
Einstellen eines Doppler-Gatters in einem Blutgefäßbereich durch Durchführen einer Bildanalyse an einem B-Modus-Bild, in dem mindestens der Blutgefäßbereich abgebildet ist;
Berechnen einer Blutflussgeschwindigkeit auf der Grundlage der Doppler-Daten im Doppler-Gatter;
Erzeugen eines Doppler-Wellenformbildes auf der Grundlage von Doppler-Daten im Doppler-Gatter;
Anzeigen des B-Modus-Bildes und des Doppler-Wellenformbildes auf einer Anzeigeeinheit; und
Anzeigen eines vergrößerten B-Modus-Bildes, in dem der Blutgefäßbereich einschließlich des Doppler-Gatters vergrößert ist, auf der Anzeigeeinheit in einem Fall, in dem sowohl das B-Modus-Bild als auch das Doppler-Wellenformbild von einem Benutzer eingefroren werden;
Erkennen einer vorderen Gefäßwand und einer hinteren Gefäßwand durch Durchführen von Bildanalyse am vergrößerten B-Modus-Bild;
Berechnen einer Querschnittsfläche eines Blutgefäßes auf der Grundlage der erkannten vorderen Gefäßwand und hinteren Gefäßwand; und
Messen einer Blutflussrate auf der Grundlage der berechneten Querschnittsfläche des Blutgefäßes und der berechneten Blutflussgeschwindigkeit.

## Revendications

1. Appareil de diagnostic à ultrasons comprenant :
une unité de définition de porte qui définit une porte Doppler dans une région de vaisseau sanguin en effectuant une analyse d'image sur une image en mode B dans laquelle au moins la région de vaisseau sanguin est imagée ;
une unité de traitement Doppler qui génère une image de forme d'onde Doppler sur la base des données Doppler dans la porte Doppler ;
une unité d'affichage qui affiche l'image en mode B et l'image de forme d'onde Doppler ; et
une unité d'agrandissement d'image qui, dans le cas où à la fois l'image en mode B et l'image de forme d'onde Doppler sont figées par un utilisateur, affiche une image en mode B agrandie dans laquelle la région de vaisseau sanguin incluant la porte Doppler est agrandie, sur l'unité d'affichage ; et
dans lequel l'unité de traitement Doppler calcule une vitesse de flux sanguin sur la base de données Doppler dans la porte Doppler, et
l'appareil de diagnostic à ultrasons comprend en outre
une unité de détection de paroi vasculaire qui détecte une paroi vasculaire antérieure et une paroi vasculaire postérieure en effectuant l'analyse d'image sur l'image en mode B agrandie ;
une unité de calcul d'aire de section transversale qui calcule une aire de section transversale d'un vaisseau sanguin sur la base de la paroi vasculaire antérieure et de la paroi vasculaire postérieure détectées par l'unité de détection de paroi vasculaire ; et
une unité de mesure de débit sanguin qui mesure un débit sanguin sur la base de l'aire de section transversale du vaisseau sanguin calculée par l'unité de calcul d'aire de section transversale et de la vitesse de flux sanguin calculée par l'unité de traitement Doppler.

2. Appareil de diagnostic à ultrasons selon la revendication 1,
dans lequel l'unité d'agrandissement d'image affiche le débit sanguin mesuré par l'unité de mesure de débit sanguin conjointement avec l'image en mode B agrandie sur l'unité d'affichage.

3. Appareil de diagnostic à ultrasons selon l'une quelconque des revendications 1 ou 2,
dans lequel l'unité d'agrandissement d'image agrandit la région de vaisseau sanguin de telle sorte qu'une position centrale de la porte Doppler soit une position centrale de l'image en mode B agrandie.

4. Appareil de diagnostic à ultrasons selon l'une quelconque des revendications 1 et 2,
dans lequel, dans un cas où la région de vaisseau sanguin à agrandir est proche d'une partie d'extrémité de l'image en mode B, l'unité d'agrandissement d'image agrandit la région de vaisseau sanguin de telle sorte que la partie d'extrémité de l'image en mode B soit une partie d'extrémité de l'image en mode B agrandie.

5. Appareil de diagnostic à ultrasons selon l'une quelconque des revendications 1 à 4,
dans lequel l'unité d'agrandissement d'image agrandit la région de vaisseau sanguin de telle sorte qu'une largeur de porte de la porte Doppler dans l'image en mode B agrandie présente une valeur prédéterminée.

6. Appareil de diagnostic à ultrasons selon la revendication 1,
dans lequel l'unité d'agrandissement d'image agrandit la région de vaisseau sanguin de telle sorte qu'une distance entre la paroi vasculaire antérieure et la paroi vasculaire postérieure dans l'image en mode B agrandie présente une valeur prédéterminée.

7. Appareil de diagnostic à ultrasons selon la revendication 1,
dans lequel l'unité d'affichage présente une région d'affichage d'image en mode B pour afficher l'image en mode B, et
l'unité d'agrandissement d'image contient une pluralité de grossissements prédéterminés qui sont différents les uns des autres, et agrandit la région de vaisseau sanguin par le grossissement maximum selon lequel la paroi vasculaire antérieure et la paroi vasculaire postérieure sont incluses dans la région d'affichage d'image en mode B, parmi la pluralité de grossissements prédéterminés.

8. Appareil de diagnostic à ultrasons selon la revendication 1,
dans lequel l'unité d'affichage présente une région d'affichage d'image en mode B pour afficher l'image en mode B et une région d'affichage d'image de forme d'onde Doppler pour afficher l'image de forme d'onde Doppler, et
l'appareil de diagnostic à ultrasons comprend en outre une unité de changement de rapport de taille qui modifie un rapport de taille de la région d'affichage d'image en mode B et de la région d'affichage d'image de forme d'onde Doppler sur la base d'une taille de l'image en mode B agrandie.

9. Appareil de diagnostic à ultrasons selon la revendication 1,
dans lequel, dans le cas où à la fois l'image en mode B et l'image de forme d'onde Doppler sont figées par l'utilisateur, le débit sanguin est automatiquement mesuré par l'unité de mesure de débit sanguin.

10. Procédé de commande d'un appareil de diagnostic à ultrasons, le procédé de commande comprenant :
la définition d'une porte Doppler dans une région de vaisseau sanguin en effectuant une analyse d'image sur une image en mode B dans laquelle au moins la région de vaisseau sanguin est imagée ;
le calcul d'une vitesse de flux sanguin sur la base des données Doppler dans la porte Doppler
la génération d'une image de forme d'onde Doppler sur la base de données Doppler dans la porte Doppler ;
l'affichage de l'image en mode B et de l'image de forme d'onde Doppler sur une unité d'affichage ; et
l'affichage d'une image en mode B agrandie dans laquelle la région de vaisseau sanguin incluant la porte Doppler est agrandie, sur l'unité d'affichage dans le cas où à la fois l'image en mode B et l'image de forme d'onde Doppler sont figées par un utilisateur ;
la détection d'une paroi vasculaire antérieure et d'une paroi vasculaire postérieure en effectuant une analyse d'image sur l'image en mode B agrandie ;
le calcul d'une aire de section transversale d'un vaisseau sanguin sur la base de la paroi vasculaire antérieure et de la paroi vasculaire postérieure détectées ; et
la mesure d'un débit sanguin sur la base de l'aire de section transversale calculée du vaisseau sanguin et de la vitesse calculée du flux sanguin.
